(19) Europäisches Patentamt
European Patent Office
Office européen des brevets

(11) Veröffentlichungsnummer: **0 037 983**
A1

# (12) EUROPÄISCHE PATENTANMELDUNG

(21) Anmeldenummer: **81102515.4**

(22) Anmeldetag: **03.04.81**

(51) Int. Cl.³: **B 01 J 29/28**, C 01 B 33/28

(30) Priorität: **16.04.80 DE 3014636**

(43) Veröffentlichungstag der Anmeldung: **21.10.81**
**Patentblatt 81/42**

(84) Benannte Vertragsstaaten: **BE DE FR GB IT NL**

(71) Anmelder: **BAYER AG, Zentralbereich Patente, Marken und Lizenzen, D-5090 Leverkusen 1, Bayerwerk (DE)**

(72) Erfinder: **Weisser, Jürgen, Dr., Südstrasse 15, D-4047 Dormagen 11 (DE)**
Erfinder: **Grolig, Johann, Dr., Heinrich-Lübke-Strasse 22, D-5090 Leverkusen 1 (DE)**
Erfinder: **Scharfe, Gerhard, Dr., Berta-von-Suttner-Strasse 69, D-5090 Leverkusen 1 (DE)**

(54) **Verfahren zur Herstellung geformter Katalysatoren auf der Basis kristalliner Aluminiumsilikate und deren Verwendung.**

(57) Die vorliegende Erfindung betrifft ein Verfahren zur Herstellung von geformten Katalysatoren mit einem minimalen Durchmesser über 1 mm und einem maximalen Durchmesser von unter 20 mm auf der Basis kristalliner Aluminiumsilikate, bei dem man Formkörper mit einem minimalen Durchmesser von über 1 mm und einem maximalen Durchmesser von unter 20 mm, die im wesentlichen aus amorphem Siliciumdioxid mit einem $SiO_2$-Gehalt von über 90 Gew.-% bestehen, mit löslichen, als Quelle für Aluminiumoxid und/oder Aluminiumhydroxid geeigneten Verbindungen des Aluminiums entsprechend einem Gehalt von 0,1 bis 20 Gew.-% Aluminiumoxid, bezogen auf die Summe der Gewichte von Aluminiumoxid plus Siliciumdioxid, umsetzt und gleichzeitig oder nacheinander mit aliphatischen $C_2$- bis $C_{24}$-Aminen oder cycloaliphatischen $C_4$- bis $C_{24}$-Aminen und/oder Ammoniumsalzen entsprechend diesen aliphatischen und/oder cycloaliphatischen Aminen und/oder den aus diesen Aminen durch Quarternierung erhältlichen quarternären Ammoniumverbindungen, in einem wäßrigen Milieu bei einem pH-Wert von größer als 8, Temperaturen von 50 bis 250 °C aussetzt und anschließend auswäscht, trocknet und bei 400 bis 600 °C calciniert. Gegenüber bekannten Verfahren zur Herstellung derartiger Katalysatoren, bei denen von löslichen oder teilweise löslichen Kieselsäuren oder Kieselsäurederivaten ausgegangen wird, haben die erfindungsgemäß hergestellten Katalysatoren den Vorteil einer einfachen Zugänglichkeit und eine Reihe weiterer Vorteile.

EP 0 037 983 A1

BAYER AKTIENGESELLSCHAFT 5090 Leverkusen, Bayerwerk
Zentralbereich
Patente, Marken und Lizenzen Gai/bc/c

# Verfahren zur Herstellung geformter Katalysatoren auf der Basis kristalliner Aluminiumsilikate und deren Verwendung

Die vorliegende Erfindung betrifft ein Verfahren zur Herstellung geformter Katalysatoren auf der Basis kristalliner Aluminiumsilikate aus amorphem Siliciumdioxid mit einem Siliciumdioxidgehalt von über 90 Gew.-%, sowie deren Verwendung als Festbettkatalysatoren.

Es ist bekannt, daß man zur Herstellung kristalliner Aluminiumsilikate, die eine katalytische Aktivität aufweisen, lösliche oder zumindest teilweise lösliche Formen von Kieselsäure einsetzen kann. Geeignet sind beispielsweise Natriumsilikat, Kieselsol oder reaktives Siliciumdioxidhydrat (siehe beispielsweise US-PS 3 702 886, US-PS 3 709 979, US-PS 4 025 571, US-PS 4 025 572, DE-OS 2 548 695 und DE-OS 2 548 697).

Als weitere Reaktionskomponenten sind lösliche Aluminiumverbindungen, z.B. Aluminate oder mineralsaure Aluminiumsalze erforderlich, die in Form einer wäßrigen Lösung und zusammen mit einem weiteren Reagenz eingesetzt werden. Bei letzterem handelt es sich in der Regel um eine organische stickstoff- oder phosphorhaltige Verbindung (siehe beispielsweise US-PS 3 702 886, US-PS 3 709 979, US-PS 4 108 881, DE-OS 2 748 278 und die Europäische Patentanmeldung mit der Veröffentlichungsnummer 0002899).

Diese Verfahren haben den entscheidenden Nachteil, daß das resultierende kristalline Aluminiumsilikat in Form einer wäßrigen Suspension anfällt, die häufig schwer zu filtrieren ist. Ebenso läßt sich der Filterkuchen sehr oft nur unter erheblichen Schwierigkeiten neutral waschen. Weiterhin ist nachteilig, daß das Reaktionsprodukt nach dem Filtrieren, Auswaschen und Calcinieren stets als mikrokristallines Pulver mit einer Partikelgröße von unter 20 µm, häufig sogar unter 10 µm erhalten wird.

In dieser Form können die kristallinen Aluminiumsilikate nicht als Festbettkatalysatoren verwendet werden. Um sie technisch einsetzen zu können, muß in nachfolgenden Arbeitsoperationen eine mechanische Formgebung erfolgen. Das kann durch Verpressen, Verpillen, Extrudieren oder durch Suspendieren, Agglomerisieren und Sprühtrocknen erfolgen. Zum Erreichen einer für die technische Anwendung notwendigen Festigkeit sind für diese Operationen Zusätze erforderlich, beispielsweise Schmier- oder Verfestigungsmittel, Binder und/oder Inertmaterialien. Diese Zusätze können jedoch einen nachteiligen Einfluß auf die Eigenschaften des erwünschten Katalysators ausüben und setzen in jedem Falle den Anteil an katalytisch aktiven Bestandteilen des Produkts herab.

Werden anstelle von löslichen oder teilweise löslichen Formen von Kieselsäure natürlich vorkommende Bodenmineralien, z.B. Smectit, als Rohstoffe für die Herstellung kristalliner und katalytisch aktiver Aluminiumsilikate verwendet, so resultieren Reaktionsprodukte, die nur zum Teil aus dem angestrebten kristallinen Silikat bestehen. So zeigt beispielsweise die US-PS 3 976 598, daß trotz Einstellung optimaler Reaktionsbedingungen hinsichtlich der Bildung eines aktiven Katalysatormaterials das Reaktionsprodukt unbefriedigend ist:

Ein für die Umwandlung von Methanol in Kohlenwasserstoffe geeignetes kristallines Aluminiumsilikat vom Typ Zeolith ZSM-5 ist nach diesem Verfahren nur in einer Konzentration von ca. 30 % im Reaktionsprodukt enthalten. Der Rest besteht hauptsächlich aus Zeolith P und ist somit für die angestrebte katalytische Verwendung nicht geeignet.

Soll aus diesem heterogenen Reaktionsprodukt ein Festbettkatalysator für die Umwandlung von Methanol in Kohlenwasserstoffe hergestellt werden, so reduziert sich der Anteil des aktiven Katalysators noch weiter, da für eine mechanische Formgebung in der Regel zumindest Bindemittel zugesetzt werden müssen.

Ein anderer, ebenfalls nachteiliger Weg zu geformten Katalysatoren ist die Verformung von Bodenmineralien mit einem hohen Aluminiumoxidgehalt, wie z.B. Ton oder Kaolin und die anschließende Umwandlung der Formkörper in ein als Katalysator, z.B. für die Methanolumwandlung, geeignetes kristallines Aluminiumsilikat.

Abgesehen von den mineralischen Verunreinigungen, die in Bodenmaterialien fast immer enthalten sind und die sich bei einer katalytischen Verwendung störend auswirken können, müssen notwendigerweise erhebliche Mengen von löslichen bzw. hochreaktiven Kieselsäurekomponenten (z.B. Wasserglas, Kieselsol, Aerosil ®) dem Ausgangsmaterial zugeführt werden, damit das Verhältnis von $SiO_2:Al_2O_3$ in einem für die Bildung des erwünschten Katalysatormaterials günstigen Bereich liegt.

Hinzu kommt, daß bei dieser Verfahrensweise das Verhältnis der Reaktionskomponenten zueinander und sogar die verwendete Wassermenge äußerst kritisch ist. Schon relativ geringe Änderungen können hier bewirken, daß anstelle des erwünschten kristallinen Aluminiumsilikats vom Typ ZSM-5 ausschließlich eine Mischung der für katalytische Zwecke wenig geeigneten Zeolithe-P und -S entsteht.

Es wurde nun ein Verfahren zur Herstellung von geformten Katalysatoren mit einem minimalen Durchmesser über 1 mm und einem maximalen Durchmesser von unter 20 mm auf der Basis kristalliner Aluminiumsilikate gefunden, das dadurch gekennzeichnet ist, daß man Formkörper mit einem minimalen Durchmesser von über 1 mm und einem maximalen Durchmesser von unter 20 mm, die im wesentlichen aus amorphem Siliciumdioxid mit einem $SiO_2$-Gehalt von über 90 Gew.-% bestehen, mit löslichen, als Quelle für Aluminiumoxid und/oder Aluminiumhydroxid geeigneten Verbindungen des Aluminiums entsprechend einem Gehalt von 0,1 bis 20 Gew.-% Aluminiumoxid, bezogen auf die Summe der Gewichte von Aluminiumoxid plus Siliciumdioxid, umsetzt und gleichzeitig oder nacheinander mit aliphatischen $C_2$- bis $C_{24}$-Aminen oder cycloaliphatischen $C_4$- bis $C_{24}$-Aminen und/oder Ammoniumsalzen entsprechend diesen aliphatischen und/oder cycloaliphatischen Aminen und/oder den aus diesen Aminen durch Quarternierung erhältlichen quarternären Ammoniumverbindungen, in einem wäßrigen Milieu, bei einem pH-Wert von größer als 8, Temperaturen von 50 bis 250°C aussetzt und anschließend auswäscht, trocknet und bei 400 bis 600°C calciniert.

Die erfindungsgemäß einzusetzenden Formkörper, die im wesentlichen aus amorphem Siliciumdioxid mit einem Siliciumdioxidgehalt von über 90 Gew.-% bestehen, können eine beliebige Gestalt innerhalb der angegebenen Abmessungen aufweisen. Insbesondere sind diejenigen geometrischen Formen geeignet, die für Festbettkatalysatoren in Frage kommen, beispielsweise Kugeln, Tabletten, Strangpreßlinge, zylindrische Extrudate, Würstchen, Tropfen, Röhrchen oder Waben. Vorzugsweise werden kugelförmige oder annähernd kugelförmige Formkörper eingesetzt, die einen Durchmesser im Bereich von 2 bis 15 mm aufweisen.
Beispielsweise lassen sich für das erfindungsgemäße Verfahren Formkörper aus amorphem Siliciumdioxid verwenden, die auf Basis synthetischer oder natürlicher Kieselsäuren hergestellt werden.

Erfindungsgemäß werden Formkörper eingesetzt, die im wesentlichen aus amorphem Siliciumdioxid bestehen, das einen Siliciumdioxidgehalt von über 90 Gew.-% aufweist. Vorzugsweise beträgt der Siliciumdioxidgehalt über 95 Gew.-%.
Zum Einsatz in das erfindungsgemäße Verfahren geeignete Formkörper sind im Handel erhältlich.
Es kann vorteilhaft sein, die Formkörper vor der erfindungsgemäßen Umsetzung mit Aluminiumverbindungen und der Behandlung mit Aminen, Ammoniumsalzen und/oder quarternären Ammoniumverbindungen in einen Zustand erhöhter

Reaktionsbereitschaft zu bringen. Dies kann beispielsweise erfolgen, indem man die Formkörper glüht und gegebenenfalls anschließend entgast. Das Glühen kann beispielsweise bei Temperaturen im Bereich von 600 bis 1100°C für beispielsweise 1 bis 6 Stunden erfolgen. Die Entgasung kann beispielsweise erfolgen, indem man die Formkörper nach dem Glühen in ein evakuierbares Gefäß einbringt und dieses evakuiert.

Es ist für das erfindungsgemäße Verfahren erforderlich, daß die Formkörper aus amorphem Siliciumdioxid mit löslichen, als Quelle für Aluminiumoxid und/oder Aluminiumhydroxid geeigneten Verbindungen des Aluminiums entsprechend einem Gehalt von 0,1 bis 20 Gew.-% Aluminiumoxid, bezogen auf die Summe der Gewichte von Siliciumdioxid plus Aluminiumoxid, umgesetzt werden. Bevorzugt wird ein Gehalt entsprechend 1 bis 10 Gew.-% Aluminiumoxid.

Die Umsetzung mit Verbindungen des Aluminiums kann z.B. so vorgenommen werden, daß man die erfindungsgemäß verwendeten Formkörper mit der Lösung einer geeigneten Aluminiumverbindung in Wasser oder einem polaren organischen Lösungsmittel tränkt und anschließend einer Wärmebehandlung unterwirft. Als polare organische Lösungsmittel können beispielsweise Alkohole, Ketone, Ether, Ester, Nitrile, Amide und/oder Sulfoxide verwendet werden. Vorzugsweise wird eine wäßrige Lösung einer Aluminiumverbindung verwendet.

Als Aluminiumverbindungen sind z.B. alle diejenigen geeignet, die in wäßriger Lösung der Hydrolyse unterliegen und in einem Gleichgewicht mit Verbindungen stehen, in denen sich mindestens eine Hydroxygruppe am Aluminiumatom befindet. Es lassen sich jedoch auch in Wasser oder organischen Lösungsmitteln neutral reagierende Aluminiumverbindungen mit gleichem Erfolg verwenden.

Geeignet sind beispielsweise die verschiedensten Aluminiumsalze und/oder Aluminiumkomplexverbindungen. Beispielsweise seien genannt: Aluminiumchlorid, -nitrat, -sulfat, -acetat, -acetylacetonat, -oxidhydrat, -fluorid, -bromid, -oxalat, Aluminiumsalze von $C_2$- bis $C_{20}$-Carbonsäuren, sowie Kaliumaluminiumsulfat, Natriumaluminiumsulfat, Aluminiumalkoholate und Alkalialuminate. Vorzugsweise verwendet man Aluminiumoxidhydrat, Aluminiumnitrat, Natriumaluminat, Aluminiumacetat und/oder Aluminiumchlorid.

In bestimmten Fällen kann es erforderlich oder vorteilhaft sein, dem Gemisch aus Aluminiumverbindung und Wasser oder Lösungsmittel Zusätze zuzufügen, um eine, gegebenenfalls schnellere, Auflösung der Aluminiumverbindung zu erreichen. So ist es z.B. beim Einsatz von Aluminiumoxidhydrat vorteilhaft, eine starke Base, z.B. Natronlauge, zuzusetzen, wodurch das i.a. schwerlösliche Aluminiumoxidhydrat in ein wesentlich besser lösliches Aluminat überführt wird.

Die Wärmebehandlung kann z.B. so vorgenommen werden, daß man das mit der Lösung einer Aluminiumverbindung getränkte Ausgangsmaterial langsam soweit erwärmt, daß das Lösungsmittel, z.B. Wasser, verdampft und die vollständig oder teilweise hydrolysierte Aluminiumverbindung im vorgeformten und erfindungsgemäß zu verwendenden Kieselsäurematerial verbleibt. Selbstverständlich kann das Lösungsmittel auch durch Evakuieren oder einfaches Trocknen an der Luft entfernt werden. Weiterhin kann das auf diese Weise mit Verbindungen des Aluminiums

umgesetzte Ausgangsmaterial einem Calcinierungsprozeß bei Temperaturen von beispielsweise 600 bis 1200°C unterworfen werden.

Die so behandelten Formkörper lassen sich in Katalysatoren auf Basis kristalliner Aluminiumsilikate umwandeln, wenn man sie in einem wäßrigen Milieu bei einem pH-Wert von größer als 8, zusammen mit Aminen, Ammoniumsalzen und/oder quartären Ammoniumverbindungen erhöhten Temperaturen aussetzt. Selbstverständlich kann auch das alkalisch-wäßrige und Stickstoffverbindungen enthaltende Milieu mit einem Zusatz von löslichen, als Quelle für Aluminiumoxid und/oder Aluminiumhydroxid geeigneten Verbindungen des Aluminiums versehen werden. Durch diese Maßnahme kann auf eine separate Umsetzung des erfindungsgemäß zu verwendenden Ausgangsmaterials mit Verbindungen des Aluminiums verzichtet werden.

Hinsichtlich der Qualität des angestrebten kristallinen Aluminiumsilikats ist es unerheblich, ob die Umsetzung mit Aluminiumverbindungen gesondert oder gleichzeitig mit dem Umwandlungs- bzw. Kristallisationsprozeß vorgenommen wird.

Wenn die Behandlung mit Aminen, Ammoniumsalzen und/oder quarternären Ammoniumverbindungen gleichzeitig mit der Behandlung mit Aluminiumverbindungen erfolgt, so kann es erforderlich sein, daß größere Mengen einer basisch reagierenden Verbindung zugesetzt werden müssen, um den für diese Behandlung notwendigen pH-Wert von größer als 8 zu erreichen. Dies ist insbesondere dann erforderlich, wenn Aluminiumverbindungen verwendet werden, die in Wasser durch Hydrolyse stark sauer reagieren, wie beispielsweise Aluminiumchlorid. Als basisch reagierende Verbindungen kommen hierfür z.B. solche in Frage, die nachstehend zur Erreichung höherer pH-Werte offenbart sind.

Die erfindungsgemäße Behandlung mit Aminen, Ammoniumsalzen und/oder quartären Ammoniumverbindungen wird vorzugsweise mit einer oder mehreren Verbindungen aus einer oder mehreren der folgenden Gruppen vorgenommen:

Monoamine der Formel

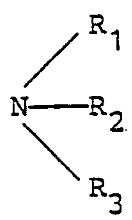

(I)

in der

$R_1$, $R_2$ und $R_3$ gleich oder verschieden sein können und für $C_2$-$C_6$-Alkyl, $C_2$-$C_6$-Hydroxyalkyl oder $C_2$-$C_6$-Aminoalkyl stehen und in der $R_1$ und/oder $R_2$ auch für Wasserstoff steht.

Diamine der Formel

$$(R_4)_2N-[C(R_5)_2]_n-N(R_6)_2 \qquad (II)$$

in der

$R_4$, $R_5$ und $R_6$ gleich oder verschieden sein können und für $C_1$-$C_2$-Alkyl, $C_1$-$C_2$-Hydroxyalkyl, $C_1$-$C_2$-Aminoalkyl oder Wasserstoff stehen und in der n für eine ganze Zahl von 2 bis 6 steht.

Monoammoniumsalze der Formel

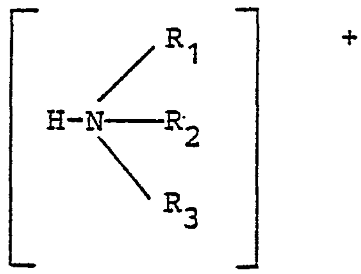

$X^-$ (III)

in der

$R_1$, $R_2$ und $R_3$ die bei Formel (I) angegebene Bedeutung haben und in der X für Cl, Br, J, $HSO_4$, $HCO_3$, $H_2PO_4$ oder $BF_4$ steht.

Monoammoniumsalze der Formel

$$[H(R_4)_2N-[C(R_5)_2]_n-N(R_6)_2]^+ \quad X^- \qquad (IV)$$

in der

$R_4$, $R_5$, $R_6$ und n die bei Formel (II) angegebene Bedeutung haben und in der X die bei Formel (III) angegebene Bedeutung hat.

Quarternäre Ammoniumverbindungen der Formel

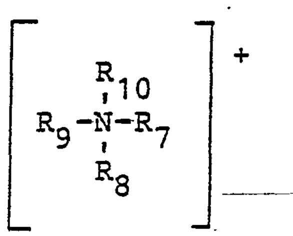

$X^-$ (V)

0037983

in der

$R_7$, $R_8$, $R_9$ und $R_{10}$ gleich oder verschieden sein können und für $C_2$-$C_4$-Alkyl, $C_2$-$C_4$-Hydroxyalkyl oder $C_2$-$C_4$-Aminoalkyl stehen,

oder in der

zwei der Reste $R_7$, $R_8$, $R_9$ und $R_{10}$ für $C_1$-$C_4$-Alkyl, $C_2$-$C_4$-Hydroxyalkyl oder $C_2$-$C_4$-Aminoalkyl und die beiden anderen Reste für $C_2$-$C_{12}$-Alkyl, Benzyl, $C_2$-$C_{12}$-Hydroxyalkyl, $C_2$-$C_{12}$-Aminoalkyl oder $C_4$-$C_8$-Cycloalkyl stehen, und

X OH ist oder die bei Formel (III) angegebene Bedeutung hat,

wobei quarternäre Ammoniumverbindungen mit mehr als insgesamt 24 C-Atomen ausgeschlossen sind.

Quarternäre Ammoniumverbindungen der Formel

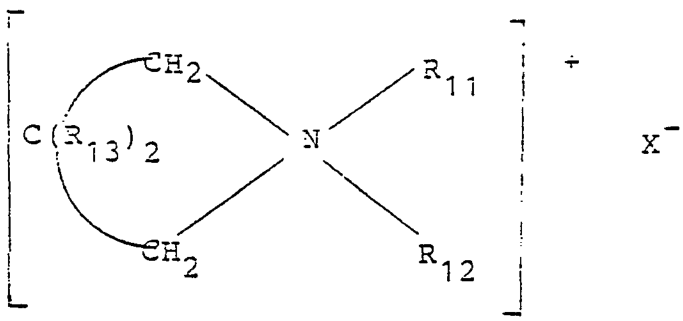

(VI)

in der

$R_{11}$ und $R_{12}$ gleich oder verschieden sein können und für $C_1$-$C_8$-Alkyl, $C_2$-$C_8$-Hydroxyalkyl oder

$C_2$-$C_8$-Aminoalkyl stehen,

$R_{13}$ für Wasserstoff oder $C_1$-$C_8$-Alkyl steht,

und X OH ist oder die bei Formel (III) angegebene Bedeutung hat,

wobei quarternäre Ammoniumverbindungen mit mehr als insgesamt 24 C-Atomen ausgeschlossen sind.

Quarternäre Ammoniumverbindungen der Formel

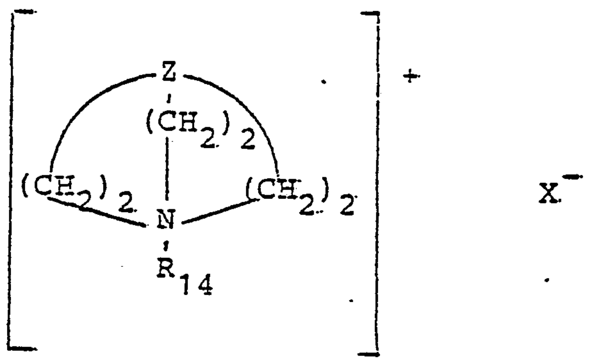

(VII)

in der

$R_{14}$ für $C_1$-$C_8$-Alkyl oder 2-Hydroxyethyl steht,

Z für Stickstoff, $-CR_{15}$ oder $-NR_{16}^{+}\ X^{-}$ steht, wobei $R_{15}$ für $C_1$-$C_8$-Alkyl steht und $R_{16}$ die gleiche Bedeutung hat, wie vorstehend für $R_{14}$ angegeben und

X OH ist oder die bei Formel (III) angegebene Bedeutung hat,

wobei quarternäre Ammoniumverbindungen mit insgesamt mehr als 24 C-Atomen ausgeschlossen sind.

Innerhalb der Verbindungen gemäß den Formeln (I) bis (VII) sind solche bevorzugt, die insgesamt 2 bis 16 C-Atome enthalten.

Besonders bevorzugt werden als Amine, Ammoniumsalze und/oder quarternäre Ammoniumverbindungen eine oder mehrere der folgenden Verbindungen eingesetzt: Tetrapropylammoniumhydroxid, -chlorid, -bromid, -jodid, Tetrabutylammoniumhydroxid, -chlorid, -bromid, -jodid, Tris-(2-hydroxyethyl)-n-propylammoniumhydroxid, -chlorid, -bromid, -jodid, sowie Tetramethylendiamin, Pentamethylendiamin, Hexamethylendiamin, n-Propylamin, n-Butylamin, Ethanolamin, Diethanolamin, Triethanolamin, wobei die genannten Amine in freier Form oder an einem Stickstoffatom protoniert und mit Chlorid, Bromid, Jodid, Hydrogensulfat, Dihydrogenphosphat, Hydrogencarbonat und/oder Tetrafluoroborat als Anion eingesetzt werden können.

Ganz besonders bevorzugt werden Tetrapropylammoniumbromid, Tetrabutylammoniumbromid und Tris-(2-hydroxyethyl)-n-propylammoniumhydroxid eingesetzt.

Die Menge, in der erfindungsgemäß Amine, Ammoniumsalze und/oder quarternäre Ammoniumverbindungen (im folgenden gemeinsam als Stickstoffverbindungen bezeichnet) eingesetzt werden können, kann in einem weiten Bereich variiert werden. Beispielsweise kann man die Stickstoffverbindungen in einer Menge von 5 bis 100 Gew.-%, bezogen auf die eingesetzten Formkörper, einsetzen. Vorzugswei-

se setzt man 10 bis 50 Gew.-% einer oder mehrerer Verbindungen aus der Gruppe der zuvor beschriebenen Stickstoffverbindungen ein, bezogen auf eingesetzte Formkörper.

Die Menge Wasser, die erfindungsgemäß einzusetzen ist, kann ebenfalls in einem weiten Bereich variiert werden. Es ist vorteilhaft, mindestens soviel Wasser einzusetzen, daß das Gemisch aus Wasser und Stickstoffverbindung(en) ausreicht, um die Poren der eingesetzten Formkörper vollständig zu füllen. Selbstverständlich können auch größere Mengen an Wasser eingesetzt werden, beispielsweise so viel, daß die eingesetzten Formkörper völlig bedeckt werden oder in einer Menge, die einem Vielfachen des Porenvolumens der eingesetzten Formkörper entspricht.

Für das erfindungsgemäße Verfahren ist es wesentlich, daß das Gemisch aus Stickstoffverbindung(en) und Wasser einen pH-Wert von größer als 8 aufweist. Vorzugsweise beträgt der pH-Wert dieses Gemisches mindestens 9, besonders bevorzugt mindestens 10.

Zur Erreichung von höheren pH-Werten kann es erforderlich sein, dem Gemisch aus Stickstoffverbindung(en) und Wasser alkalisch reagierende Stoffe zuzusetzen. Außer stark alkalisch reagierenden Verbindungen aus der Gruppe der zuvor beschriebenen Stickstoffverbindungen kommen hierfür auch die verschiedensten organischen und/oder anorganischen Verbindungen mit ausreichender Alkalität

in Frage. Beispiele für derartige organische und anorganische Verbindungen sind Amine, wie Methyl-, Dimethyl-, Trimethyl-, Ethyl-, Diethyl-, Triethyl-, Methylethyl-, Propyl- und Butylamin, Anilin, Methyl-, Ethyl-, Dimethyl-, Diethyl-, Methylethylanilin, Chinolin und Pyridin; anorganische Oxide und Hydroxide, wie Alkali- und Erdalkalioxide und Alkali- und Erdalkalihydroxide, insbesondere Natrium-, Kalium-, Lithium-, Magnesium-, Calcium- und Bariumoxid oder -hydroxid; Salze aus starken Basen und schwachen Säuren, wie Carbonate, Phosphate, Silikate, Acetate, Fluoride, Stannite, Stannate, Borate, Wolframate, Molybdate, Sulfide, Sulfite von Natrium, Kalium, Lithium, Magnesium, Calcium und Barium.
Vorzugsweise kommen als Zusätze solche in Frage, die in Wasser gut löslich sind und mit den Stickstoffverbindungen und den eingesetzten Formkörpern nicht in unerwünschter Weise reagieren.

Auch aus anderen Gründen kann es vorteilhaft sein, dem Gemisch aus Stickstoffverbindung(en) und Wasser Zusätze zuzufügen. So wurde beispielsweise festgestellt, daß bei Zusätzen von Alkalicarbonaten, Alkaliphosphaten, Alkalifluoriden, Alkalisulfiden, Alkalicarboxylaten, Alkalisilikaten und/oder Alkaliwolframaten geformte Katalysatoren auf der Basis kristalliner Aluminiumsilikate erhalten werden können, die eine besonders gute Bruchhärte aufweisen. Dies ist insbesondere bei Zusätzen von Alkaliphosphaten, Alkalicarbonaten, Alkalisilikaten, Alkalisulfiden und/oder Alkaliwolframaten der Fall.

Die Zusätze zur Erhöhung des pH-Wertes und/oder zur Erzielung guter Bruchhärten können in weiten Grenzen variiert werden. Im allgemeinen sind Zusätze in einer Menge ausreichend, die bezogen auf die eingesetzten Formkörper 0,01 bis 10, vorzugsweise 0,1 bis 5 Gew.-% Hydroxidionen freisetzen können. Falls gleichzeitig mit der Behandlung mit Aminen, Ammoniumsalzen und/oder quarternären Ammoniumverbindungen die Umsetzung mit Aluminiumverbindungen erfolgt und durch Hydrolyse sauer reagierende Aluminiumverbindungen eingesetzt werden, können auch größere Mengen solcher Zusätze vorteilhaft oder erforderlich sein. Wesentlich ist in diesem Zusammenhang der gewünschte pH-Wert und nicht die maximal stöchiometrisch mögliche Menge freisetzbarer Hydroxidionen.

Die Stickstoffverbindung(en), das Wasser und die einzusetzenden Formkörper, sowie die gegebenenfalls einzusetzenden Zusätze können in beliebiger Reihenfolge zusammengegeben werden. Im allgemeinen geht man so vor, daß man aus Wasser, Stickstoffverbindung(en) und gegebenenfalls einem oder mehreren Zusätzen eine Lösung oder Suspension herstellt und die einzusetzenden Formkörper damit übergießt. Ebenfalls können die für das erfindungsgemäße Verfahren erforderlichen Aluminiumverbindungen der Lösung oder Suspension aus Stickstoffverbindung(en), Wasser und gegebenenfalls Zusätzen zugefügt werden.

Nach dem Zusammengeben von Wasser, Stickstoffverbindung(en), einzusetzenden Formkörpern, Aluminiumverbindungen und gegebenenfalls Zusätzen wird das dabei gebildete Reaktionsgemisch Temperaturen im Bereich 50 bis 250°C, vorzugsweise im Bereich 80 bis 180°C, ausgesetzt. Dabei wird das amorphe Ausgangsmaterial unter Beibehaltung seiner äußeren Form zum größten Teil oder vollständig in kristallines Aluminiumsilikat überführt. Die Umwandlungsgeschwindigkeit hängt im wesentlichen von der Beschaffenheit des Ausgangsmaterials und der Temperatur ab. Man kann z.B. durch Debye-Scherrer (Röntgenbeugungs-)Analyse von Proben den jeweils erreichten Kristallinitätsgrad feststellen. Mit steigender Reaktionstemperatur nimmt die notwendige Behandlungszeit stark ab. Im allgemeinen können gute Ergebnisse erhalten werden, wenn man das Reaktionsgemisch beispielsweise 20 bis 60 Stunden bei ca. 180°C oder etwa 80 bis 150 Stunden bei ca. 120°C oder etwa 350 bis 500 Stunden bei ca. 80°C beläßt. In einzelnen Fällen können jedoch auch von diesen Richtwerten mehr oder weniger stark abweichende Behandlungszeiten notwendig sein.

Wenn die Behandlung bei Temperaturen vorgenommen werden soll, bei denen Teile des Reaktionsgemisches unter Normaldruck flüchtig sind, so wird zweckmäßigerweise in einem druckbeständigen, verschließbaren Gefäß gearbeitet. Es ist nicht notwendig, während der Behandlung zu rühren.

Wenn durch die Behandlung der gewünschte Kristallinitätsgrad erreicht worden ist, so wird das feste Reaktionsprodukt zunächst von der gegebenenfalls vorhandenen überschüssigen wäßrigen Phase abgetrennt, beispielsweise durch eine mechanische Abtrennung wie Dekantieren oder Filtrieren. Die abgetrennte wäßrige Phase kann, gegebenenfalls nach Ergänzung der verbrauchten Bestandteile, wiederverwendet werden. Es ist dann, auch wenn nach der Behandlung keine mechanisch abtrennbare wäßrige Phase vorhanden war, noch erforderlich, das feste Reaktionsprodukt bei 400 bis 600°C zu calcinieren. Diese Calcinierung wird durchgeführt, nachdem das feste Reaktionsprodukt mit Wasser neutral gewaschen und getrocknet worden ist.

Man erhält so geformtes, zum größten Teil oder vollständig kristallines Aluminiumsilikat, das meist schon eine gewisse katalytische Aktivität aufweist. In vielen Fällen kann die katalytische Aktivität noch verbessert werden.

Es ist deshalb vorteilhaft, das so erhaltene geformte, zum größten Teil oder vollständig kristalline Aluminiumsilikat in an sich bekannter Weise einem Ionenaustausch mit Ammoniumionen oder Wasserstoffionen zu unterziehen und es anschließend nochmals zu calcinieren.

So behandelte Produkte weisen im allgemeinen eine gute katalytische Aktivität auf. Sie eignen sich insbesondere als Katalysatoren für die Umwandlung von Methanol und/oder Dimethylether in Kohlenwasserstoffe, als Crackkontakte, für die Alkylierung, Disproportionierung und/oder Isomerisierung von Aromaten und für die Aromatisierung von Olefinen. Vorzugsweise werden derartige Produkte für die Umwandlung von Methanol und/oder Dimethylether in Kohlenwasserstoffe, für die Alkylierung, Disproportionierung und/oder Isomerisierung von Aromaten oder für die Aromatisierung von Olefinen verwendet.

Es kann auch vorteilhaft sein, insbesondere um hochaktive Katalysatoren für spezielle Zwecke zu erhalten, während oder nach dem Ionenaustausch weitere, die katalytischen Eigenschaften verbessernde Komponenten in das Aluminiumsilikat einzubringen. Als derartige Komponenten kommen beispielsweise Schwermetalle, Edelmetalle, Nichtmetallverbindungen und/oder Verbindungen der Seltenen Erden in Frage, die nach an sich bekannten Techniken eingebracht werden können.

Bei einer beispielhaften technischen Ausführungsform des erfindungsgemäßen Verfahrens kann wie folgt verfahren werden:

Kugelförmiges amorphes Siliciumdioxid mit etwa 5 mm Partikeldurchmesser und einem Siliciumdioxidgehalt von über 90 Gew.-% wird bei einer Temperatur von über 800°C geglüht und danach im Vakuum entgast. Das so vorbehandelte Ausgangsmaterial wird mit einem Gemisch aus wäßriger Natronlauge, gelöster Aluminiumverbindung und einer oder mehreren Stickstoffverbindungen versetzt, wobei die Menge dieses Gemisches einem Vielfachen des Porenvolumens des Ausgangsmaterials entspricht. Die Natronlauge kann beispielsweise 0,1 bis 5 Gew.-% Hydroxidionen (bezogen auf eingesetztes Siliciumdioxid) enthalten, gegebenenfalls auch mehr. Als Aluminiumverbindungen werden vorzugsweise Natriumaluminat, Aluminiumsulfat, Aluminiumchlorid, Natriumaluminiumsulfat, Kaliumaluminiumsulfat, Aluminiumacetat oder Aluminiumfluorid in einer Konzentration entsprechend 0,1 bis 20 Gew.-% Aluminiumoxid, bezogen auf die Summe der Gewichte von Aluminiumoxid plus Siliciumdioxid, verwendet. Als Stickstoffverbindungen können beispielsweise Tetrapropylammoniumhalogenide, Tetrabutylammoniumhalogenide, Hexamethylendiamin, Ethanolamin, Propylamin, Butylamin oder entsprechende quarternäre Ammoniumverbindungen in einer Menge von 5 bis 100 Gew.-% (bezogen auf eingesetztes Ausgangsmaterial) eingesetzt werden. In einem alkali- und druckbeständigen Reaktionsgefäß wird dann bei 100 bis 150°C im Verlaufe von 10 bis 25 Tagen das amorphe Siliciumdioxid in weitgehend kristallines Aluminiumsilikat überführt. Nach Beendigung der Kristallisation wird die überschüssige

wäßrige Phase mechanisch abgetrennt. Durch Waschen mit Wasser entfernt man überschüssiges Alkali und trocknet das Reaktionsprodukt. Anschließend wird dieses bei 400 bis 600°C calciniert, dann so lange mit einer wäßrigen Ammoniumsalzlösung behandelt, bis der überwiegende Teil der im Reaktionsprodukt vorhandenen austauschbaren Natriumionen durch Ammoniumionen ersetzt ist. Dann wird nochmals getrocknet und calciniert und so ein Katalysator erhalten, der direkt in Festbettreaktoren eingefüllt werden kann, in denen beispielsweise Methanol und/oder Dimethylether in Kohlenwasserstoffe umgewandelt werden. Der Katalysator kann auch für die Aromatisierung von Olefinen, für das katalytische Cracken, für die Alkylierung und Disproportionierung von Aromaten oder für Adsorptionen eingesetzt werden. Der Katalysator ist, gegebenenfalls nach weiterer Modifizierung, auch für andere katalytische Reaktionen einsetzbar.

Die erfindungsgemäß hergestellten Katalysatoren sind wesentlich einfacher zugänglich als entsprechende Katalysatoren, die gemäß dem Stand der Technik aus löslichen oder teilweise löslichen Kieselsäuren oder Kieselsäurederivaten hergestellt werden. Die erfindungsgemäß hergestellten Katalysatoren haben eine solche mechanische Stabilität (pro Formkörper), daß sie auch bei technischem Dauereinsatz nicht zerfallen und somit der Druckverlust in Festbettreaktoren beim technischen Einsatz dieser Katalysatoren auch bei längeren Laufzeiten gering ist. Selbst thermische Regenerierungen, durch die im Verlauf der katalytischen Umwandlungen entstehende Koksbeläge oberhalb 500°C im Luftstrom abgebrannt werden können, beeinträchtigen die mechanischen Eigenschaften der Katalysatoren nur unwesentlich.

Weiterhin ist beim erfindungsgemäßen Verfahren vorteilhaft, daß die einzusetzende Wassermenge in weiten Grenzen variierbar ist.

Es ist ausgesprochen überraschend, daß die erfindungsgemäße Behandlung von Formkörpern aus amorphem Siliciumdioxid nicht zum Zerfall der Formkörper bzw. zur Bildung von mechanisch wenig stabilen Formkörpern führt, denn bei der Behandlung mit basischen Mitteln konnte die Bildung von löslichen Silikaten bzw. Aluminaten erwartet werden und damit der Zerfall der eingesetzten Formkörper zumindest schon bei geringer mechanischer Belastung. Überraschenderweise besitzen die erfindungsgemäß hergestellten Katalysatoren eine gute mechanische Stabilität (Bruchhärte), trotz der Verwendung eines basischen und deshalb aggressiven Reaktionsmediums, das beispielsweise mehrere 100 Stunden bei erhöhter Temperatur einwirkt. Insbesondere war bei der Verwendung relativ stark basischer Verbindungen aus der Gruppe der Alkali- und Erdalkalihydroxide, der Alkalicarbonate, Alkaliphosphate, Alkalifluoride, Alkalisulfide, Alkalicarboxylate, Alkalisilikate und Alkaliwolframate zu erwarten, daß das Einsatzmaterial so angegriffen wird, daß es nicht zur Bildung formstabiler, für einen technischen Einsatz in Festbettreaktoren geeigneter Katalysatoren kommt.

Das erfindungsgemäße Verfahren und die Verwendung der dabei erhältlichen Produkte wird durch die folgenden Beispiele erläutert, ohne die Erfindung auf diese Beispiele einzuschränken.

## Beispiele

### Beispiel 1

Dieses Beispiel betrifft die Herstellung eines für die Umwandlung von Methanol und/oder Dimethylether in Kohlenwasserstoffe geeigneten Katalysators.

1 kg eines kugelförmigen Katalysatorträgermaterials mit einem Durchmesser von 3 bis 5 mm auf Basis amorpher Kieselsäure wurde durch Evakuieren entgast und mit einer Lösung bestehend aus 104,4 g Aluminiumoxidhydrat (47 % $Al_2O_3$), 80 g Natriumhydroxid und 2000 ml Wasser getränkt. Die Mischung wurde zur Trockne eingedampft und im Vakuum bei 150°C getrocknet.

Das so behandelte Material wurde nach Abkühlung mit einer Lösung bestehend aus 300 g Tetrapropylammoniumbromid, 30 g Trinatriumphosphat und 1200 ml Wasser versetzt und bei einer Temperatur von 97°C belassen. Nach 18 Tagen wies das ursprünglich amorphe Ausgangsmaterial eine gute Kristallinität auf, was durch eine Debye-Scherrer-Aufnahme festgestellt wurde.

Die Reaktionslösung wurde dann abdekantiert und das in kristallines Aluminiumsilikat umgewandelte Material solange mit Wasser gewaschen, bis die ablaufende Waschlösung einen pH-Wert von 7 bis 8 aufwies. Das gewaschene Produkt wurde im Vakuum bei 120°C getrocknet und zur Entfernung organischer Bestandteile bei einer Temperatur von 550°C 4 Stunden lang calciniert.

Es resultierte die Natriumform eines kristallinen Sili-

0037983

kates mit einer Zusammensetzung entsprechend den molaren Verhältnissen:

$$Na_2O:Al_2O_3:SiO_2 \text{ wie } 1,2:1:39,2.$$

Durch Behandlung mit einem Überschuß an verdünnter Ammoniumchloridlösung wurde die Ammoniumform erhalten, die bei einer Temperatur von 500°C thermisch zersetzt wurde.

Zur Überprüfung der katalytischen Aktivität wurden 30 ml des so hergestellten kugelförmigen Festbettkatalysators in einen elektrisch beheizten und mit einer Vorheizzone, sowie mit Einlaßvorrichtungen für Stickstoff und Methanol versehenen Quarzreaktor gefüllt und bei einer Temperatur von 370°C ohne Anwendung von Druck mit Methanol in Berührung gebracht. Bei einer Kontaktbelastung von 800 g Methanol pro l Katalysator und pro Stunde wurde folgendes Ergebnis erhalten:

Methanolumsatz: 96 Gew.-%.

Produktzusammensetzung ohne Berücksichtigung des Reaktionswassers (Gew.-%):

| | |
|---|---|
| $H_2$: | 0,1 |
| $CH_4$: | 0,7 |
| $C_2H_6$: | 0,1 |
| $C_2H_4$: | 6,9 |
| $C_3H_8$: | 2,3 |
| $C_3H_6$: | 15,5 |
| $C_4H_{10}$: | 5,0 |
| $C_4H_8$: | 8,0 |
| $C_5^+$: | 40,4 |
| $CH_3$-O-$CH_3$: | 8,0 |
| $C_6^+$-Aromaten: | 13,0 |

Sowohl in frischem Zustand als auch nach Versuchsende zeigte der Katalysator eine Bruchhärte, die ihn für einen technischen Einsatz geeignet erscheinen läßt.

Beispiel 2

Dieses Beispiel zeigt, daß sich der nach Beispiel 1 hergestellte Katalysator auch für die Umwandlung von Dimethylether in Kohlenwasserstoffe eignet.

An einem gemäß Beispiel 1 hergestellten Katalysator wurde bei 350°C und einer Kontaktbelastung von 300 g Dimethylether pro l Katalysator und pro Stunde ohne Anwendung von Druck Dimethylether zu 94 % in ein Gemisch aus Wasser, sowie flüssigen und gasförmigen Kohlenwasserstoffen umgewandelt. Ohne Berücksichtigung von Reaktionswasser und nicht umgesetztem Dimethylether wurde folgende Produktverteilung erhalten (Angaben in Gew.-%):

| | |
|---|---|
| $C_6^+$ - Aromaten | 22,5 |
| $H_2$: | 0,1 |
| $CH_4$: | 0,8 |
| $C_2H_6$: | 0,9 |
| $C_2H_4$: | 3,5 |
| $C_3H_8$: | 5,0 |
| $C_3H_6$: | 3,9 |
| $C_4H_{10}$: | 8,5 |
| $C_4H_8$: | 5,0 |
| $C_5^+$: | 49,8 |

Beispiel 3

Dieses Beispiel zeigt die Eignung des nach Beispiel 1 hergestellten Katalysators für die Aromatisierung von Ethylen.

50 ml des nach Beispiel 1 hergestellten Katalysators wurden in einen elektrisch beheizten Reaktor gefüllt und bei 350°C mit einem aus gleichen Teilen Stickstoff und Ethylen bestehenden Gasstrom (Strömungsgeschwindigkeit 500 ml/h) in Berührung gebracht.

Neben einer nahezu ethylenfreien Gasphase wurde ein flüssiges Reaktionsgemisch der folgenden Zusammensetzung erhalten (Angaben in Gew.-%):

| | |
|---|---|
| $C_5^+$-Aliphaten: | 22,5 |
| Benzol: | 3,4 |
| Toluol: | 15,0 |
| $C_8$-Aromaten: | 20,1 |
| Hochsieder: | 39,0 |

Beispiel 4

Dieses Beispiel zeigt die Eignung des nach Beispiel 1 hergestellten Katalysators für die Disproportionierung von Aromaten.

Toluol wurde verdampft und bei einer Temperatur von 540°C über den gemäß Beispiel 1 hergestellten Katalysator geleitet. Bei einer Kontaktbelastung von 1 Vol.-Teil Toluol (flüssig) pro Vol.-Teil Katalysator und pro Stunde und einem Druck von 1,0 bar konnte ein Reaktionsprodukt mit folgender Zusammensetzung erhalten werden (Angaben in Gew.-%):

| | |
|---|---|
| Benzol: | 5,8 |
| Toluol: | 86,0 |
| $C_8^+$-Aromaten: | 8,2 |

Beispiel 5

Dieses Beispiel verdeutlicht, daß die Umwandlung eines geformten Siliciumdioxids in kristallines Aluminiumsilikat auch dann in der erwünschten Weise verläuft, wenn die Umsetzung des Ausgangsmaterials mit Verbindungen des Aluminiums gleichzeitig mit der Wärme- und Basenbehandlung durchgeführt wird.

100 g eines Kieselsäuretropfkontaktes vom Typ DU 591/3/6 wurden durch Glühen bei 900°C vorbehandelt und mit einer Mischung bestehend aus 10,63 g Aluminiumoxidhydrat (47 % $Al_2O_3$), 11,2 g Kaliumhydroxid, 13,3 g Tetrapropylammoniumbromid, 5 g Ethanolamin und 150 ml Wasser getränkt.

Die Mischung wurde in einen Stahlautoklaven überführt und bei einer Temperatur von 112°C belassen. Nach einer Zeit von 16 Tagen wies eine Probe des Reaktionsproduktes eine gute Kristallinität auf, wie durch eine Debye-Scherrer-Aufnahme festgestellt werden konnte.

Die Aufarbeitung und Aktivierung des Reaktionsproduktes, das sich in der Alkaliform aus 3,19 Gew.-% $K_2O$, 2,99 Gew.-% $Al_2O_3$ und 89,91 Gew.-% $SiO_2$ (Differenz zu 100 % = im wesentlichen Feuchtigkeit) zusammensetzte, wurde

wie im Beispiel 1 beschrieben durchgeführt.

Zur Überprüfung der katalytischen Aktivität wurden 30 ml des Katalysators in einen elektrisch beheizten Festbettreaktor gefüllt und bei einer Temperatur von 370°C ohne Anwendung von Druck mit Methanol in Berührung gebracht.

Bei einer Kontaktbelastung von 791 g Methanol pro l Katalysator und pro Stunde wurde folgendes Ergebnis erhalten (ohne Berücksichtigung des Reaktionswassers):

| Versuchsdauer (Stunden) | 132 | 156 | 180 |
|---|---|---|---|
| Umsatz (Gew.-%) | 33,9 | 30,6 | 29,0 |
| C-Selektivität zu $C_2$- bis $C_4$-Olefinen (%) | 54,7 | 74,3 | 74,6 |
| Produktverteilung (Gew.-%) | | | |
| $CO$, $CO_2$ | 0,4 | 0,7 | 0,7 |
| $H_2$ | 0,2 | 0,3 | 0,2 |
| $CH_4$ | 1,5 | 2,3 | 2,2 |
| $C_2H_6$ | 0,1 | 0,2 | 0,1 |
| $C_2H_4$ | 10,6 | 13,2 | 10,0 |
| $C_3H_8$ | 2,2 | 2,5 | 1,8 |
| $C_3H_6$ | 15,6 | 19,0 | 15,7 |
| $C_4H_{10}$ | 5,1 | 6,0 | 4,6 |
| $C_4H_8$ | 6,1 | 7,0 | 6,0 |
| $C_5^+$ | 11,2 | 3,0 | 2,2 |
| $CH_3OCH_3$ | 40,2 | 45,8 | 56,4 |
| Aromaten | 6,7 | - | - |

Das nach diesem Beispiel hergestellte und hinsichtlich seiner katalytischen Aktivität charakterisierte kristalline Aluminiumsilikat zeichnete sich durch eine gute mechanische Festigkeit aus, die auch im Verlauf des in diesem Beispiel beschriebenen Dauerversuchs nahezu unverändert erhalten blieb.

## Beispiel 6

Dieses Beispiel zeigt die Umwandlung eines geformten und amorphen Siliciumdioxids in kristallines Aluminiumsilikat in Gegenwart von Hexamethylendiamin und Alkalicarbonat.

43,4 g eines handelsüblichen, nahezu aluminiumfreien Kieselsäureträgermaterials in Perlenform (Durchmesser 3 bis 6 mm) wurden mit einer wäßrigen Lösung, bestehend aus 37 g $Al(NO_3)_3 \cdot 9H_2O$ und 100 ml $H_2O$ bei Raumtemperatur getränkt. Nach Abdampfen des überschüssigen Wassers und Trocknung bei 120°C und 20 mbar wurde das so mit Aluminiumnitrat behandelte Material 4 Stunden bei 900°C calciniert. Anschließend wurde bei Raumtemperatur durch Evakuieren eine Entgasung vorgenommen und danach mit 140 ml einer 25 %igen wäßrigen Hexamethylendiaminlösung getränkt. Nach Zugabe von 7,1 g $Na_2CO_3 \cdot 10H_2O$ und Durchmischung wurde der Ansatz in einen Stahlautoklav überführt und 180 Stunden bei einer Temperatur von 180°C belassen.

Das Reaktionsprodukt wies nach Auswaschen, Trocknen und Calcinieren die für die kristallinen Aluminiumsilikat-Zeolithe ZSM-5 bzw. ZSM-11 charakteristischsten Röntgenbeugungslinien auf. Weiterhin zeigten die Formkörper nach dem Kristallisationsprozeß eine ungewöhnlich hohe und für technische Zwecke voll ausreichende Bruchhärte und Formbeständigkeit.

Beispiel 7

Dieses Beispiel betrifft die Verwendung eines Phosphat-Organoammoniumhydroxid-Gemisches bei der Umwandlung eines geformten und amorphen Siliciumdioxids in kristallines Aluminiumsilikat.

43,4 g eines handelsüblichen, nahezu aluminiumfreien Kieselsäureträgermaterials in Perlenform (Durchmesser 3 bis 6 mm) wurde mit einer wäßrigen Lösung, bestehend aus 37 g $Al(NO_3)_3 \cdot 9H_2O$ und 100 ml $H_2O$ bei Raumtemperatur getränkt. Nach Abdampfen des überschüssigen Wassers und Trocknung bei 120°C und 20 mbar wurde das so mit Aluminiumnitrat behandelte Material 4 Stunden bei 900°C calciniert.

Anschließend wurde bei Raumtemperatur durch Evakuieren eine Entgasung vorgenommen und danach mit 140 ml einer wäßrigen Lösung aus Tetrapropylammoniumhydroxid (15 Gew.-%) und Trinatriumphosphat (10 Gew.-%) getränkt.

Die Mischung wurde in einen Stahlautoklav überführt und 160 Stunden bei einer Temperatur von 150°C belassen.

Das Reaktionsprodukt wies nach Auswaschen, Trocknen und Calcinieren die für die kristallinen Aluminiumsilikat-Zeolithe ZSM-5 bzw. ZSM-11 charakteristischsten Röntgenbeugungslinien auf.

0037983

Weiterhin zeigten die Formkörper nach dem Kristallisationsprozeß eine hohe und für technische Zwecke voll ausreichende Bruchhärte und Formbeständigkeit.

Beispiel 8 (Vergleichsbeispiel)

Dieses Vergleichsbeispiel verdeutlicht die technischen Probleme, die auftreten können, wenn ein nach konventioneller Synthese hergestelltes, kristallines Aluminiumsilikat als Festbettkatalysator verwendet werden soll.

Unter Verwendung der Ausgangsmaterialien Aluminiumoxid, Kieselsol, Natronlauge, Wasser und Monoethanolamin wurde gemäß der europäischen Patentanmeldung mit der Veröffentlichungs-Nr. 0002899, Beispiel 1, ein kristallines Aluminiumsilikat hergestellt.

Bei dem Reaktionsprodukt handelte es sich um staubförmiges Material, das aufgrund seiner mikrokristallinen Beschaffenheit nicht als Festbettkatalysator verwendet werden konnte.

Dieses Material wurde mit einem Blauton als Bindemittel im Verhältnis 5:1 versetzt und mit Wasser zu einer extrudierbaren Paste verarbeitet. Es resultierte nach Verformung, Trocknung, Calcinierung, Ionenaustausch ($Na^+$ gegen $H^+$) und nochmaliger Trocknung und Calcinierung ein geformter Katalysator von bröckeliger Beschaffenheit, d.h. relativ hoher Empfindlichkeit gegen mechanische Beanspruchung.

Mit diesem Katalysator konnten bei einer Temperatur von 370°C, einem Druck von 1 bar und einer Methanolzufuhr von 791 g pro l Katalysator und pro Stunde folgende Versuchsergebnisse erhalten werden:

| Versuchsdauer (Stunden) | 4 | 8 | 12 | 24 | 36 |
|---|---|---|---|---|---|
| Methanolumsatz (Gew.-%) | 99,9 | 99,7 | 97,7 | 64,9 | 36,4 |
| Produktzusammensetzung in Gew.-% (ohne Berücksichtigung des Reaktionswassers) | | | | | |
| $C_6^+$-Aromaten | 35,9 | 34,8 | 31,0 | 18,9 | 0,0 |
| $CO$, $H_2$, $CH_4$, $CO_2$ | 8,3 | 3,0 | 2,5 | 1,6 | 2,4 |
| $C_2H_6$ | 1,3 | 0,5 | 0,5 | 0,1 | 0,1 |
| $C_2H_4$ | 1,3 | 2,6 | 5,7 | 0,1 | 0,2 |
| $C_3H_8$ | 18,0 | 12,5 | 9,9 | 0,0 | 0,0 |
| $C_3H_6$ | 1,9 | 2,8 | 5,3 | 0,1 | 0,1 |
| $C_4H_{10}$ | 19,6 | 20,0 | 17,3 | 0,0 | 0,0 |
| $C_4H_8$ | 5,0 | 6,5 | 7,0 | 0,2 | 0,0 |
| $C_5^+$ | 6,6 | 15,5 | 14,4 | 5,1 | 0,0 |
| $CH_3$-O-$CH_3$ | 2,1 | 1,9 | 6,4 | 73,9 | 97,2 |

Dieses Versuchsergebnis zeigt, daß dieser auf konventionelle Weise hergestellte Festbettkatalysator zwar eine hohe Anfangsaktivität besitzt, aber schon nach einer Versuchszeit von weniger als 24 Stunden überwiegend Dimethylether produziert. Nach einer Versuchsdauer von 36 Stunden sind außer $C_1$-Verbindungen und Spuren leichter Olefine keine der erwünschten Kohlenwasserstoffe (Olefine, Aromaten, $C_5^+$) im Reaktionsprodukt mehr enthalten.

Demgegenüber zeigen die erfindungsgemäß hergestellten Katalysatoren neben der mechanischen Beständigkeit eine wesentlich länger andauernde Aktivität, wie z.B. aus Beispiel 5 hervorgeht.

Patentansprüche

1) Verfahren zur Herstellung von geformten Katalysatoren mit einem minimalen Durchmesser über 1 mm und einem maximalen Durchmesser von unter 20 mm auf der Basis kristalliner Aluminiumsilikate, dadurch gekennzeichnet, daß man Formkörper mit einem minimalen Durchmesser von über 1 mm und einem maximalen Durchmesser von unter 20 mm, die im wesentlichen aus amorphem Siliciumdioxid mit einem $SiO_2$-Gehalt von über 90 Gew.-% bestehen, mit löslichen, als Quelle für Aluminiumoxid und/oder Aluminiumhydroxid geeigneten Verbindungen des Aluminiums entsprechend einem Gehalt von 0,1 bis 20 Gew.-% Aluminiumoxid, bezogen auf die Summe der Gewichte von Aluminiumoxid plus Siliciumdioxid, umsetzt und gleichzeitig oder nacheinander mit aliphatischen $C_2$- bis $C_{24}$-Aminen oder cycloaliphatischen $C_4$- bis $C_{24}$-Aminen und/oder Ammoniumsalzen entsprechend diesen aliphatischen und/oder cycloaliphatischen Aminen und/oder den aus diesen Aminen durch Quarternierung erhältlichen quarternären Ammoniumverbindungen, in einem wäßrigen Milieu bei einem pH-Wert von größer als 8, Temperaturen von 50 bis 250°C aussetzt und anschließend auswäscht, trocknet und bei 400 bis 600°C calciniert.

2) Verfahren nach Anspruch 1, dadurch gekennzeichnet, daß man Formkörper, die einen Siliciumdioxidgehalt von über 95 Gew.-% aufweisen, einsetzt.

3) Verfahren nach Ansprüchen 1 und 2, dadurch gekennzeichnet, daß man Aluminiumsalze und/oder Aluminiumkomplexverbindungen einsetzt.

4) Verfahren nach Ansprüchen 1 bis 3, dadurch gekennzeichnet, daß man die Formkörper mit der Lösung einer Aluminiumverbindung in Wasser oder einem polaren organischen Lösungsmittel tränkt und einer Wärmebehandlung unterwirft und danach mit aliphatischen $C_2$- bis $C_{24}$-Aminen oder cycloaliphatischen $C_4$- bis $C_{24}$-Aminen und/oder Ammoniumsalzen entsprechend diesen aliphatischen und/oder cycloaliphatischen Aminen und/oder den aus diesen Aminen durch Quarternierung erhältlichen quarternären Ammoniumverbindungen, in einem wäßrigen Milieu, bei einem pH-Wert von größer als 8, Temperaturen von 50 bis 250°C aussetzt und anschließend auswäscht, trocknet und bei 400 bis 600°C calciniert.

5) Verfahren nach Ansprüchen 1 bis 4, dadurch gekennzeichnet, daß man dem wäßrigen Milieu Amine, anorganische Oxide, anorganische Hydroxide und/oder Salze aus starken Basen und schwachen Säuren zusetzt.

6) Verfahren nach Ansprüchen 1 bis 5, dadurch gekennzeichnet, daß man dem wäßrigen Milieu Alkalicarbonate, Alkaliphosphate, Alkalifluoride, Alkalisulfide, Alkalicarboxylate, Alkalisilikate und/oder Alkaliwolframate zusetzt.

7) Verwendung von gemäß den Ansprüchen 1 bis 6 hergestellten Katalysatoren als Festbettkatalysatoren für die Umwandlung von Methanol und/oder Dimethylether in Kohlenwasserstoffe, als Crack-Kontakte, für die Alkylierung, Disproportionierung und/oder Isomerisierung von Aromaten oder für die Aromatisierung von Olefinen.

0037983

Europäisches Patentamt

**EUROPÄISCHER RECHERCHENBERICHT**

Nummer der Anmeldung
EP 81 10 2515

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 2 643 928 (IMPERIAL CHEMICAL INDUSTRIES)<br>* Ansprüche 5 und 7; Seite 10, Zeile 9 bis Seite 11, Zeile 6; Seite 12, Zeilen 16-30; Beispiele 1 und 5 *<br>--- | 1,3, 5,7 |
| | US - A - 3 422 033 (H.D. BALLARD et al.)<br>* Insgesamt *<br>--- | 1,5 |
| A | US - A - 3 515 682 (W.H. FLANK et al.)<br>* Spalte 6, Zeilen 27-41 *<br>--- | 1 |
| A | US - A - 3 348 911 (E. MICKALKO) | |
| A | US - A - 4 091 007 (F.G. DWYER et al.) | |
| A | GB - A - 1 099 073 (UNIVERSAL OIL PRODUCTS Co.)<br>& DE - A - 1 567 861 | |
| A | EP - A - 0 007 126 (UNION CARBIDE CORPORATION)<br>--------- | |

KLASSIFIKATION DER ANMELDUNG (Int. Cl.)

B 01 J 29/28
C 01 B 33/28

RECHERCHIERTE SACHGEBIETE (Int. Cl.)

B 01 J 29/28
C 01 B 33/28
B 01 J 29/06

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsatze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 20.07.1981 | CECCHINI |